# EUROPEAN PATENT SPECIFICATION

(11) **EP 3 917 585 B1**
(45) Date of publication and mention of the grant of the patent: **02.12.2026**
(21) Application number: 19817665.3
(22) Date of filing: 09.12.2019
(51) Int. Cl.: A61L 27/16, A61L 27/34, A61L 27/50, A61L 27/56

(54) **ELECTROSPUN COVER LAYER FOR MEDICAL IMPLANTS**
ELEKTROGESPONNENE DECKSCHICHT FÜR MEDIZINISCHE IMPLANTATE
COUCHE DE REVÊTEMENT ÉLECTROFILÉE POUR IMPLANTS MÉDICAUX

(30) Priority: 29.01.2019 US 201962798227 P
(43) Date of publication of application: 08.12.2021
(73) Proprietor: Xeltis AG, 8008 Zurich (CH)
(72) Inventor: LOPES, Susana Amorim, 8152 Zurich (CH); VAN DER BURGT, Vivian, 5616 PE Eindhoven (NL); BAELUS, Betty, 5642 EC Eindhoven (NL); COX, Martijn Antonius Johannes, 6021 RC Budel (NL)
(74) Representative: FRKelly
(86) International application number: PCT/EP2019/084147
(87) International publication number: WO 2020/156711

(56) References cited:
- EP-B1- 2 968 674
- US-A1- 2013 268 062
- US-A1- 2016 317 295
- US-A1- 2017 340 460

## Description

### FIELD OF THE INVENTION

This invention is defined by the appended claims and relates to devices and methods to cover medical implants for enhanced durability and wear reduction.

### BACKGROUND OF THE INVENTION

The durability of bioprosthetic heart valves is unfortunately limited due to abrasion and stress on the valves. An important aspect in maximizing the durability of bioprosthetic heart valves is the reduction of areas with abrasion and stress concentrations.

Nowadays, heart valve struts are often covered with additional synthetic fabric, usually PTFE or polyester (one or more layers) to reduce abrasion-related wear from direct contact to bare metal or other hard parts. The synthetic fabric then serves as an anchor point for porcine or pericardial tissue attachment. This solution is not ideal since durability problems are only reduced and the materials used can lead to infections in the valve prosthesis.

EP 2 968 674 discloses thin, biocompatible, high-strength, composite materials that are suitable for use in a valve for regulating blood flow direction. In one aspect, the composite material maintains flexibility in high-cycle flexural applications, making it particularly applicable to high-flex implants such as a prosthetic heart valve leaflet. The composite material includes a porous polymer membrane and an elastomer, wherein the elastomer fills substantially all of the pores of the porous polymer membrane.

The present invention focuses on a different technique to cover medical implants for enhanced durability and wear reduction.

### SUMMARY OF THE INVENTION

The present invention is defined by the claims and provides a medical implant that distinguishes a medical implant support structure, an electrospun cover layer covering at least a portion of the medical implant support structure, and an electrospun medical implant layer covering the electrospun cover layer such that the electrospun cover layer is an in-between layer in between the portion of the medical implant support structure and the electrospun medical implant layer and therewith preventing direct contact for the medical implant support structure with the electrospun medical implant layer and ensuring that the electrospun cover layer is in direct contact with the electrospun medical implant layer.

The electrospun cover layer is a bioabsorbable porous electrospun cover layer. The bioabsorbable porous electrospun cover layer is capable of being absorbed and replaced by natural tissue due to ingrowth of cells and nutrient into the pores of the bioabsorbable porous electrospun cover layer. The porous electrospun cover layer has a pore size distribution of 5 to 50 micrometers.

The electrospun medical implant layer is a bioabsorbable porous electrospun medical implant layer. The bioabsorbable porous electrospun medical implant layer is also capable of being absorbed and replaced by natural tissue due to ingrowth of cells and nutrient into the pores of the bioabsorbable porous electrospun medical implant layer. The porous electrospun medical implant layer has a pore size distribution of 5 to 50 micrometers.

In one example, the electrospun medical implant layer is a heart valve or leaflet.

In the present invention, the medical implant support structure is a metal wire support structure with posts for a heart valve. The electrospun cover layer could cover one or more posts. Disclosed is that the electrospun cover layer could be directly electrospun onto or over the portion of the medical implant support structure. In the present invention, the electrospun cover layer is a closed tube slid over the portion of the medical implant support structure. The electrospun cover layer could be stitched onto the portion of the medical implant support structure. The electrospun cover layer could also be glued to the portion of the medical implant support structure.

### BRIEF DESCRIPTION OF THE DRAWINGS

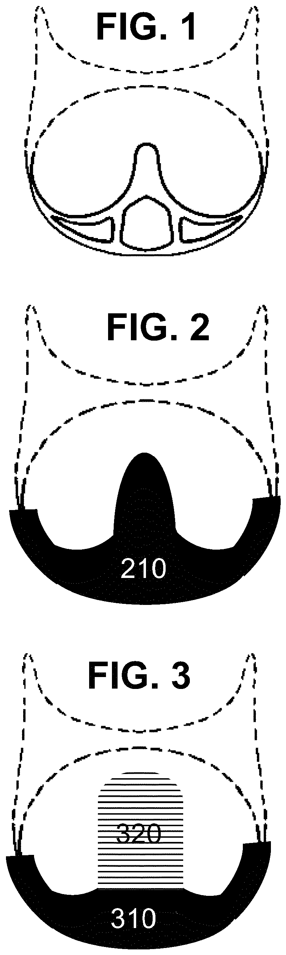

- **FIG. 1**: shows a metallic wire (support structure) for a heart valve with three posts.
- **FIG. 2**: shows according to the present invention the metallic wire of **FIG. 1** with added, over a portion of the metallic wire, an electrospun cover layer **210.** It is noted that the figure only shows a portion of the metal wire frame being covered with the electrospun cover layer for clarity purposes. The invention is not limited to embodiments where only a portion is being covered by the electrospun cover layer as the objective of the invention is to cover the metallic wire at locations of the metallic wire where abrasion is a problem. In one embodiment, only the struts/posts could be covered, where in another embodiment most if not all of the metallic wire could be covered with the electrospun cover layer/material.
- **FIG. 3**: shows the metallic wire of **FIG. 1** with electrospun cover layer **210** according to the present invention. In addition to the electrospun cover layer **210** covering the portion of the metal wire frame and according to the present invention, the covered post of the metal wire frame is further covered with electrospun layer **310** (which is shown by the additional dashed curvature over the post). It is noted that the figure only shows one post being covered with the second electrospun layer. It is further noted that each post could have such an additional electrospun layer in addition to the electrospun covered layer. In one example (not shown), the electrospun layer could be an electrospun leaflet or heart valve that is placed over the metallic wire and therewith also placed over the electrospun cover layer **210,** whereby the electrospun cover layer **210** serves as the in-between layer, in between the metallic wire and the electrospun leaflet or heart valve.

### DETAILED DESCRIPTION

The present invention provides a medical implant for a heart valve with an electrospun cover layer in between a support structure of the medial implant and an electrospun layer, whereby the electrospun cover layer is intended for enhanced durability and wear reduction. Specifically, in one embodiment, the invention provides a heart valve metallic wire structure (support structure, **FIG. 1**) with the electrospun cover layer (**210,** **FIG. 2**) covering at least a portion of the metallic wire structure and situated in between the heart valve metallic wire structure and an electrospun heart valve or leaflet (**310,** **FIG. 3**). For clarity reasons the electrospun heart valve or leaflet is not shown and, in this example, is only shown or represented by **310.**

At stated *supra,* the electrospun cover layer covers at least a portion of the support structure. In the example of the heart valve, would be the heart valve strut being a typical area where abrasion occurs. As a result of the electrospun cover layer over the support structure, the electrospun heart valve is then no longer in direct contact with the support structure. Instead, the electrospun heart valve is now in direct contact with the electrospun cover layer.

The inventors have found that the abrasion between two electrospun layers is significantly less than the abrasion between other non-electrospun structures and the electrospun polymer according to this invention. It was therefore concluded that the electrospun cover layer helps to reduce abrasion-related wear of an electrospun leaflet or heart valve, resulting in increased durability.

More generally speaking the electrospun cover layer could be a cover layer over a metal support structure or even over a synthetic layer (not part of the invention) to the effect that the electrospun cover layer becomes the in-between layer between a (metal) support structure and an electrospun layer such as the heart valve or leaflet, or even a synthetic layer or animal-derived tissue layer (not part of the invention) positioned over the electrospun cover layer.

Support structures could be directly covered or encapsulated into layer(s) of electrospun material, in particular from bioabsorbable polymers. This would stimulate the Endogenous Tissue Restoration (ETR) process and prevent leaflet wear at the same time. The electrospun cover layer is then of porous nature which is important as it makes the electrospun cover layer bioabsorbable and therewith capable of being absorbed and replaced by natural tissue due to ingrowth of cells and nutrients into pores of the electrospun cover layer. The pore size distribution of the pores is 5 to 50 micrometers. The electrospun cover layer could therewith also referred to as bioabsorbable porous electrospun cover layer.

The design of the electrospun cover layer varies per application and are provided as small tubes that fit over a strut/post of a metallic wire support structure for a heart valve. Since closed tubes are used they do not have any wear, abrasion or breaking points and would cover the struts in a perfect way. In addition, electrospun material is less bulky and reduces the overall size of the final device. Another design option is in sheets (not part of the invention).

In the embodiment of a tube, electrospinning could be performed directly onto a wire instead of a bigger mandrel. This technique produces very fine tubes with a very small inner diameter.

Useful embodiments could be directly electrospun on top of the struts, sliding of the electrospun cover material, in particular, tubes on top of the struts or over the struts, gluing of the material to the struts, stitching (with suture wires) of the material (e.g. sheets or tubes) directly on the struts.

The electrospun material referenced in this invention may include the ureido-pyrimidinone (UPy) quadruple hydrogen-bonding motif (pioneered by Sijbesma (1997), Science 278, 1601-1604) and a polymer backbone, for example selected from the group of biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

The same result may be obtained with alternative, non-supramolecular polymers, if properties are carefully selected and material processed to ensure required surface characteristics. These polymers may comprise biodegradable or non-biodegradable polyesters, polyurethanes, polycarbonates, poly(orthoesters), polyphosphoesters, polyanhydrides, polyphosphazenes, polyhydroxyalkanoates, polyvinylalcohol, polypropylenefumarate. Examples of polyesters are polycaprolactone, poly(L-lactide), poly(DL-lactide), poly(valerolactone), polyglycolide, polydioxanone, and their copolyesters. Examples of polycarbonates are poly(trimethylenecarbonate), poly(dimethyltrimethylenecarbonate), poly(hexamethylene carbonate).

### Experimental Data

The inventors studied ePTFE fabric and an electrospun cover layer which are both polymeric fibrous structures, for which one would expect similar abrasive characteristics. However, and surprisingly, the inventors found that the electrospun cover layer showed significantly improved results when compared to ePTFE fabric as shown below in **Table 1.**

**Table 1**

| **Valve #** | **Leaflet design** | **Cover on frame** | **Cycle count AWT [Million cycles]** |
|---|---|---|---|
| XSAV-158 | A | ePTFE fabric | 3.2 |
| XSAV-159 | A | ePTFE fabric | 0.67 |
| XSAV-162 | B | Electrospun Cover (small diameter tubes) | 35 |
| XSAV-163 | B | Electrospun Cover (small diameter tubes) | 26 |

Valves with ePTFE fabric covered frame (XSAV-158 and XSAV-159) failed very early in an Accelerated Wear Test (AWT), while valves with the electrospun cover layer covering the synthetic fabric frame (XSAV-162 and XSAV-163) are running much longer. This showed a great and unexpected increase in durability when covering the frame with electrospun cover layer.

## Claims

1. A medical implant for a heart valve, comprising:
(a) a metal wire support structure with posts for the heart valve;
(b) a bioabsorbable porous electrospun cover layer, in the form of a closed tube slid over at least a portion of the medical implant support structure to cover the portion; and
(c) a bioabsorbable porous electrospun medical implant layer covering the electrospun cover layer such that the electrospun cover layer is an in-between layer in between the portion of the medical implant support structure and the electrospun medical implant layer and therewith preventing direct contact for the medical implant support structure with the electrospun medical implant layer and ensuring that the electrospun cover layer is in direct contact with the electrospun medical implant layer.

2. The medical implant as set forth in claim 1, wherein the bioabsorbable porous electrospun cover layer is capable of being absorbed and replaced by natural tissue due to ingrowth of cells and nutrient into the pores of the bioabsorbable porous electrospun cover layer.

3. The medical implant as set forth in claim 1, wherein the electrospun cover layer is porous with a pore size distribution of 5 to 50 micrometers.

4. The medical implant as set forth in claim 1, wherein the bioabsorbable porous electrospun medical implant layer is capable of being absorbed and replaced by natural tissue due to ingrowth of cells and nutrient into the pores of the bioabsorbable porous electrospun medical implant layer.

5. The medical implant as set forth in claim 1, wherein the electrospun medical implant layer is porous with a pore size distribution of 5 to 50 micrometers.

6. The medical implant as set forth in claim 1, wherein the electrospun medical implant layer is a heart valve or leaflet.

7. The medical implant as set forth in claim 1, wherein the electrospun cover layer covers one or more posts.

8. The medical implant as set forth in claim 1, wherein the electrospun cover layer is directly electrospun onto or over the portion of the medical implant support structure.

## Patentansprüche

1. Medizinisches Implantat für eine Herzklappe, umfassend:
(a) eine Metalldraht-Trägerstruktur mit Pfosten für die Herzklappe;
(b) eine bioabsorbierbare, poröse, elektrogesponnene Deckschicht in Form eines geschlossenen Schlauchs, der über mindestens einen Teil der medizinischen Implantat-Trägerstruktur geschoben ist, um den Teil zu bedecken; und
(c) eine bioabsorbierbare, poröse, elektrogesponnene medizinische Implantatschicht, die die elektrogesponnene Deckschicht bedeckt, sodass die elektrogesponnene Deckschicht eine Zwischenschicht zwischen dem Teil der medizinischen Implantat-Trägerstruktur und der elektrogesponnenen medizinischen Implantatschicht ist und damit direkten Kontakt für die medizinische Implantat-Trägerstruktur mit der elektrogesponnenen medizinischen Implantatschicht verhindert und sicherstellt, dass die elektrogesponnene Deckschicht in direktem Kontakt mit der elektrogesponnenen medizinischen Implantatschicht steht.

2. Medizinisches Implantat nach Anspruch 1, wobei die bioabsorbierbare, poröse, elektrogesponnene Deckschicht aufgrund des Einwachsens von Zellen und Nährstoff in die Poren der bioabsorbierbaren, porösen, elektrogesponnenen Deckschicht absorbiert und durch natürliches Gewebe ersetzt werden kann.

3. Medizinisches Implantat nach Anspruch 1, wobei die elektrogesponnene Deckschicht porös ist mit einer Porengrößenverteilung von 5 bis 50 Mikrometern.

4. Medizinisches Implantat nach Anspruch 1, wobei die bioabsorbierbare, poröse, elektrogesponnene medizinische Implantatschicht aufgrund des Einwachsens von Zellen und Nährstoff in die Poren der bioabsorbierbaren, porösen, elektrogesponnenen medizinischen Implantatschicht absorbiert und durch natürliches Gewebe ersetzt werden kann.

5. Medizinisches Implantat nach Anspruch 1, wobei die elektrogesponnene medizinische Implantatschicht porös ist mit einer Porengrößenverteilung von 5 bis 50 Mikrometern.

6. Medizinisches Implantat nach Anspruch 1, wobei die elektrogesponnene medizinische Implantatschicht eine Herzklappe oder ein Segel ist.

7. Medizinisches Implantat nach Anspruch 1, wobei die elektrogesponnene Deckschicht einen oder mehrere Pfosten bedeckt.

8. Medizinisches Implantat nach Anspruch 1, wobei die elektrogesponnene Deckschicht direkt auf oder über den Teil der medizinischen Implantat-Trägerstruktur elektrogesponnen ist.

## Revendications

1. Implant médical pour une valve cardiaque, comprenant :
(a) structure de support en fil métallique avec des montants pour la valve cardiaque ;
(b) une couche de revêtement électrofilée poreuse bioabsorbable, sous la forme d'un tube fermé glissé sur au moins une partie de la structure de support d'implant médical pour recouvrir la partie ; et
(c) une couche d'implant médical électrofilée poreuse bioabsorbable recouvrant la couche de revêtement électrofilée de sorte que la couche de revêtement électrofilée est une couche intermédiaire entre la partie de la structure de support d'implant médical et la couche d'implant médical électrofilée et empêchant de ce fait le contact direct de la structure de support d'implant médical avec la couche d'implant médical électrofilée et assurant que la couche de revêtement électrofilée est en contact direct avec la couche d'implant médical électrofilée.

2. Implant médical selon la revendication 1, dans lequel la couche de revêtement électrofilée poreuse bioabsorbable est capable d'être absorbée et remplacée par du tissu naturel en raison de la croissance de cellules et de nutriments dans les pores de la couche de revêtement électrofilée poreuse bioabsorbable.

3. Implant médical selon la revendication 1, dans lequel la couche de revêtement électrofilée est poreuse avec une distribution de taille de pores de 5 à 50 micromètres.

4. Implant médical selon la revendication 1, dans lequel la couche d'implant médical électrofilée poreuse bioabsorbable est capable d'être absorbée et remplacée par du tissu naturel en raison de la croissance de cellules et de nutriments dans les pores de la couche d'implant médical électrofilée poreuse bioabsorbable.

5. Implant médical selon la revendication 1, dans lequel la couche d'implant médical électrofilée est poreuse avec une distribution de taille de pores de 5 à 50 micromètres.

6. Implant médical selon la revendication 1, dans lequel la couche d'implant médical électrofilée est une valve ou un feuillet cardiaque.

7. Implant médical selon la revendication 1, dans lequel la couche de revêtement électrofilée recouvre un ou plusieurs montants.

8. Implant médical selon la revendication 1, dans lequel la couche de revêtement électrofilée est directement électrofilée sur ou au-dessus de la partie de la structure de support d'implant médical.
